# EUROPÄISCHE PATENTSCHRIFT

(11) **EP 1 387 701 B1**
(45) Veröffentlichungstag und Bekanntmachung des Hinweises auf die Patenterteilung: **27.10.2004**
(21) Anmeldenummer: 02735057.8
(22) Anmeldetag: 03.05.2002
(51) Int. Cl.: A61K 51/10, A61K 47/48, A61K 41/00, A61K 49/00

(54) **IMMUNKONJUGATE AUS EIDOTTER-ANTIKÖRPERN (IGY), DEREN KONFEKTIONIERUNG UND ANWENDUNG IN DIAGNOSTIK UND THERAPIE**
IMMUNOCONJUGATES MADE OF EGG-YOLK ANTIBODIES (IGY), PRODUCTION AND USE THEREOF IN DIAGNOSES AND THERAPY
CONJUGUES IMMUNITAIRES COMPOSES D'ANTICORPS DE JAUNE D'OEUF (IGY), FABRICATION ET UTILISATION DANS LE DIAGNOSTIC ET LA THERAPIE

(30) Priorität: 15.05.2001 DE 10123505
(43) Veröffentlichungstag der Anmeldung: 11.02.2004
(73) Patentinhaber: Bergter, Wolfgang, Dr., 30625 Hannover (DE); Kobilke, Hartmut, Dr., 14797 Damsdorf (DE); Sroka, Joachim, 14797 Damsdorf (DE)
(72) Erfinder: Bergter, Wolfgang, Dr., 30625 Hannover (DE); Kobilke, Hartmut, Dr., 14797 Damsdorf (DE); Sroka, Joachim, 14797 Damsdorf (DE)
(74) Vertreter: Heitsch, Wolfgang
(86) Internationale Anmeldenummer: PCT/DE2002/001606
(87) Internationale Veröffentlichungsnummer: WO 2002/092136

(56) Entgegenhaltungen:
- WO-A-95/02612
- DE-A- 19 737 453
- KIM, HYUN-OCK ET AL: "Reusability of Avidin - Biotinylated Immunoglobulin Y Columns in Immunoaffinity Chromatography" ANALYTICAL BIOCHEMISTRY (1999), 268(2), 383-397 , XP002905439
- DATABASE CA [Online] CHEMICAL ABSTRACTS SERVICE, COLUMBUS, OHIO, US; YANG, TAO-LAN ET AL: "Studies on programmed cell death of cancer induced by IgY - ricin A" retrieved from STN Database accession no. 130:108365 XP002242617 & YICHUAN XUEBAO (1998), 25(5), 392-397 ,
- DATABASE CA [Online] CHEMICAL ABSTRACTS SERVICE, COLUMBUS, OHIO, US; LIU, LIANRUI ET AL: "Anti-stomach cancer immunoglobulin Y- ricin A conjugate for cancer targeting therapy" retrieved from STN Database accession no. 122:64353 XP002242618 & CN 1 085 910 A (GENETICS INSTITUTE, PEOP. REP. CHINA) 27. April 1994 (1994-04-27)
- LIU, L. R. ET AL: "Studies on antibody- IgY against stomach cancer" RECENT ADV. CHEM. MOL. BIOL. CANCER RES., INT. SYMP. (1993), MEETING DATE 1991, 195-200. EDITOR(S): DAI, QIANHUAN;ARMOUR, MARGARET-ANN; ZHENG, QINGYING. PUBLISHER: SCIENCE PRESS, BEIJING, PEOP. REP. CHINA. , XP008017611
- LEE S C ET AL: "PURIFICATION OF HUMAN ALPHA-ANTIPLASMIN WITH CHICKEN IGY SPECIFIC TO ITS CARBOXY-TERMINAL PEPTIDE" PREPARATIVE BIOCHEMISTRY, NEW YORK, NY, US, Bd. 27, Nr. 4, 1997, Seiten 227-237, XP002918319
- DATABASE CA [Online] CHEMICAL ABSTRACTS SERVICE, COLUMBUS, OHIO, US; HORIE, NORIKO ET AL: "Sustained-release preparations containing emulsified compositions" retrieved from STN Database accession no. 132:227451 XP002242619 & JP 2000 080027 A (TAIYO KAGAKU CO., LTD., JAPAN) 21. März 2000 (2000-03-21)
- CIPOLLA A ET AL: "Campylobacter fetus diagnosis: direct immunofluorescence comparing chicken IgY and rabbit IgG conjugates." ALTEX: ALTERNATIVEN ZU TIEREXPERIMENTEN. GERMANY 2001, Bd. 18, Nr. 3, 2001, Seiten 165-170, XP008017617 ISSN: 0946-7785

## Beschreibung

Die vorliegende Erfindung betrifft IgY-Konjugate aus Eidotter-Antikörpern (IgY) und deren Konfektionierung zu Diagnostika oder Therapeutika nach den Merkmalen der Ansprüche 1 und 13.

Zum besseren Verständnis der Erfindung werden die in der Anmeldudg vorkommenden Fachtermini hier näher definiert und sollen im Rahmen dieser Anmeldung keine andere Bedeutung haben:

### "Spezifiziert Pathogenfreie Hühner" (kurz "SPF-Hühner"):

Es werden solche Tiere verstanden, die nach den Richtlinien von Europäischer Pharmakopoe und DAB10 von SPF-Muttenieren abstammen und vom ersten Lebenstag an unter SPF-Bedingungen gehalten werden. Diese Tiere sind frei von menschen- und geflügelpathogenen Erregern und deren Antikörpern.

### "IgY" (engl.: immunoglobulin yolk):

Hier werden Immunglobuline bezeichnet, die aus dem Eidotter von Geflügeleiern extrahiert werden und die dem IgG im Serum der Hühner entsprechen. Diese aviären Immunglobuline unterscheiden sich vom IgG des Säugers strukturell vor allem durch ihr höheres Molekulargewicht infolge der größeren Zahl konstanter Domänen im Fc-Fragment.

### "Spezifisches IgY":

Ist hier definiert als der Anteil des gesamten IgY, der das zur Immunisierung verwendete Antigen erkennt, während unspezifisches IgY den Anteil des gesamten IgY bezeichnet, der unabhängig von der Immunisierung durch den Kontakt der Tiere mit antigen wirkenden Stoffen oder apathogenen und pathogenen Erregern gebildet wird.

### "Intakte Antikörper":

Es sollen Immunglobuline verstanden werden, die nicht fragmentiert sind, die also ein Fc-Fragment und zwei Fab-Fragmente besitzen, wobei Fc die konstanten Regionen der schweren Ketten umfasst und Fab die variablen Regionen der schweren und leichten Ketten.

### "IgY-Fragmente":

Mit "IgY-Fragmenten" sind hier Fab-Fragmente aviärer Immunglobuline gemeint, die keinen oder nur noch einen geringen Rest des Fc-Fragments besitzen. Dabei sollen hier nachfolgend unter dem Begriff "Fab" zur Vereinfachung auch Fragmente wie F(ab)₂ verstanden werden.

### "Fab-Konstrukt":

Der Begriff "Fab-Konstrukt" soll synthetische Gebilde aus zwei oder mehr verschiedenen Fab-Fragmenten (bi- oder trivalente Konstrukte etc.) bezeichnen, die eine oder mehre unterschiedliche antigene Determinanten erkennen (mono-, bi- oder trispezifische Konstrukte usw.) und somit eine oder mehrere Funktionen erfüllen können, d. h. mono-, bi- oder trifunktional wirken.

### "Antigene Determinante":

Dies ist diejenige Molekülstruktur auf einem Antigen, die von einem Antikörper spezifisch erkannt wird (auch "Epitop" genannt).

### "IgY-Präparation":

Es sollen die aus dem Eidotter extrahierten und ggf. gereinigten Eidotter-Antikörper (IgY), IgY-Fragmente, Fab-Konstrukte oder chimäre Eidotter-Antikörper verstanden werden.

### "Signalstoff":

Hierunter sind alle Stoffe zu verstehen, die eine analytische, visuelle oder bildgebende Beurteilung eines pathologischen oder physiologischen Befundes ermöglichen.

### "Wirkstoff":

Es werden hierunter alle Stoffe bezeichnet, die therapeutisch nutzbar sind, z. B. Enzyme, Antibiotika, Virostatika und Zellgifte wie Toxine oder Radionuklide. Dabei ist nicht ausgeschlossen, dass der Wirkstoff zugleich die Qualität eines Signalstoffs erfüllt.

### "Prodrug":

Hiermit seien alle Stoffe bezeichnet, die aus einer inaktiven Vorstufe im Körper vor Ort in einen aktiven Wirkstoff umgewandelt werden.

### "Boosterung" und "Auffrischungsimmunisierung":

Im Zusammenhang mit der Immunisierung von SPF-Hühnem wird von diesen beiden Begriffen die Rede sein. Die Boosterungen dienen dazu, den maximalen IgY-Titer zu erreichen. Unter Auffrischungsimmunisierung wird die erneute Antigenexposition verstanden, durch die der maximale IgY-Titer bis zum Ende der Legeperiode aufrecht erhalten wird.

Bekannt sind aus dem Stand der Technik Immunkonjugate auf der Basis von monoklonalen Antikörpern aus der Maus (murine MAK) oder vom Menschen (humane MAK). Eine Vielzahl von Methoden wurden etabliert, um Signalstoffe (Farbstoffe, Fluorochrome, diagnostische Radionuklide) oder Wirkstoffe (z. B. Toxine, therapeutische Radionuklide, Photosensibilisatoren, Prodrugs) an diese Antikörper zu konjugieren. Damit lassen sich Untersuchungen wie ELISA, RIA, Fluoreszenzmikroskopie, Duchflußzytometrie, Szintigraphien, SPECT (engl.: single photon emission computed tomography), PET (Positronen-Emissionstomogra- phie) und Photoimmundiagnostik beziehungsweise Behandlungen wie Radioimmuntherapie, Immuntoxintherapie oder photodynamische Therapie durchführen.

Zu den Nachteilen der monoklonalen Antikörpertechnologie gehören vor allem die Monospezifität der Antikörper, sowie die relativ aufwendige und kostenintensive Entwicklung und Produktion der monoklonalen Antikörper. Die Massenproduktion muss im Allgemeinen in großen Fermentem erfolgen, da die Produktion im Aszites von Mäusen inzwischen in vielen Ländern weitgehend untersagt ist.

Polyklonale Immunseren von Säugetieren (Schaf, Pferd, Schwein usw.) und immunisierten/infizierten Spendern (z. B. aus dem Blut von HIV-Infizierten mit hohem p24-Antikörper-Titer) spielen bei verschiedenen diagnostischen Verfahren und passiven Immuntherapien eine Rolle. Die Polyklonalität dieser Immunseren ist unter dem Gesichtspunkt eines besseren Targetings komplexer Antigene sinnvoll. Immunseren von Säugern oder konventionellen Hühnern enthalten jedoch ein relativ schlecht definiertes polyspezifisches Antikörper-Gemisch, wobei die gewünschten spezifischen Antikörper den geringsten Anteil bilden, während unspezifische und somit unerwünschte Antikörper den Hauptanteil ausmachen. Die Gewinnung von Immunseren aus Säugetieren ist unter dem Gesichtspunkt des Tierschutzes kaum vertretbar und daher in mehreren Ländern bereits weitgehend verboten. Immunseren aus humanen Blutspenden zu gewinnen ist teuer, keineswegs risikolos (Übertragung von Erregern wie HIV, HCV und möglicherweise Prionen), ethisch problematisch und daher für eine breite Anwendung nicht praktikabel.

Polyklonale Antikörper aviären Ursprungs, unter anderem Eidotter-Antikörper (IgY), sind im Stand der Technik ebenfalls bekannt. IgY wird heute von verschiedenen Herstellern für die in vitro-Diagnostik als Alternative zu Immunseren von Säugern oder monoklonalen Antikörpern angeboten. In der Fachliteratur finden sich auch Beispiele dafür, wie Eidotter-Antikörper mit Biotin, FITC oder Meerrettich- (Horse Radish) Peroxidase (POD) gekoppelt werden können, um sie für die in vitro-Diagnostik nutzbar zu machen (SCHADE et al, 2001 [1]).

Bekannt ist auch die orale Verwendung aviärer Antikörper aus herkömmlichen Hühnern. Der parenterale Einsatz in der Humanmedizin wurde bisher weitgehend ausgeschlossen, einerseits weil IgY bei Säugern aufgrund des phylogenetischen Abstands weder an Komplement-Faktor C1 noch an Fc-Rezeptoren bindet und somit mit dem Abwehrsystem des Säugers nicht effektiv interagieren kann und andererseits weil IgY allergische Reaktionen auslösen kann. Hinzu kommt, dass die Herstellung von IgY aus dem Eidotter konventioneller Hühner weniger effizient ist und bezüglich Reinheit und Affinität zu qualitativ minderwertigen Produkten führt. Der Grund dafür ist, dass herkömmliche Hühner zahlreiche unspezifische Antikörper gegen andere Erreger bilden und die Antwort auf das Zielimmunogen daher relativ schwach ausfällt und nur kurz anhält. Daher liegt der Anteil von spezifischem IgY am Gesamt-IgY bei der Immunisierung konventioneller Hühner weit unter 10 % (US 4,550,019; HANSEN et al., 1998 [2]; BOUHOURS et al 1998 [3]; GASSMANN et al, 1990 [4]).

Den nächsten Stand der Technik beschreibt die DE 195 04 755, in der es um eine passive Immuntherapie der HIV-Infektion mit IgY geht. Das IgY ist gegen die HIV-core-Antigene p24 und p17 aus spezifiziert pathogenfreien (SPF-) Hühnern gerichtet. Passive Immuntherapien mit humanen Immunseren sind ebenfalls bekannt. Im Vergleich zu Immunseren und monoklonalen Antikörpern lässt sich IgY aber vergleichsweise leicht und preiswert herstellen. SPF-Hühner legen unter dem in Beispiel 2 beschriebenen Immunisierungsprotokoll nach Grundimmunisierung und Boosterung bis zum 16. Lebensmonat wöchentlich 5 - 7 Eier. Dabei hat sich gezeigt, dass aus den 250 - 350 Eiern eines SPF-Huhns rund 15.000 mg spezifisches IgY gewonnen werden kann, weil erfahrungsgemäß I Eidotter mindestens 50 mg IgY der gewünschten Spezifität enthält. Darüber hinaus sind die Belastungen von SPF-Hühnem wegen der für diese Tiere vorgeschriebene artgerechte Tierhaltung wesentlich geringer als bei der herkömmlichen Mast- und Legehuhn-Haltung. Dadurch entfallen die üblichen aktiven Immunisierungen gegen bis zu 19 wirtschaftlich bedeutsame Infektionskrankheiten wie sie bei der herkömmlichen Hühnerzucht üblich sind, darunter zwei Pflichtimpfungen. Diese sind ja dafür verantwortlich, dass die IgY-Präparationen konventioneller Hühner minderwertig sind und dass sich die Isolierung spezifischer IgY erschwert und verteuert. Die Haltung von SPF-Hühnem erfolgt frei von human- und geflügelpathogenen Keimen. Zum Ausschluss solcher Keime werden regelmäßige Futter-, Wasser- und Immunstatus-Kontrollen durchgeführt. Auf den Zusatz von Antibiotika im Futter wird verzichtet, um den SPF-Status der Tiere nicht zu kaschieren.

Aus medizinischer Sicht liegen die Vorteile von IgY allgemein in der Erkennung mehrerer antigener Determinanten durch die spezifischen Antikörper, was ein besseres Targeting des Zielantigens gewährleistet, und in der Tatsache, dass es sich um Antikörper aus Geflügel handelt, dessen Immunsystem sich hinsichtlich der Antigen-Erkennung von dem des Säugetiers und des Menschen unterscheidet. Dies verbessert ebenfalls das Targeting des Zielantigens, denn das aviäre Immunsystem erkennt (phylogenetisch bedingt) andere antigene Determinanten als das menschliche humane Immunsystem. Daher müssen aviäre Antikörper in vivo weniger mit den natürlich vorhandenen Antikörpern des Patienten konkurrieren um Epitope zu erkennen. Darüber hinaus kann sich der vermeintliche Nachteil, dass aviäre Antikörper weder mit dem menschlichen Komplement-System noch mit Fc-Rezeptoren, Protein A oder Protein G reagieren in vielen Fäl-Ien als unwesentlich oder gar als vorteilhaft erweisen.

Aufgabe der vorliegenden Erfindung ist es deshalb, alternative Produkte zu herkömmlichen lmmunkonjugaten zu entwickeln und bereitzustellen, die sich als Diagnostika oder Therapeutika eignen und dabei den monoklonalen Antikörpern und Immunseren aus Säugetieren, sowie den IgY-Präparationen aus herkömmlichen Hühnern hinsichtlich Reinheit und Antigen-Targeting überlegen sind. Die Bildung von humanen anti-IgY-Antikörpern soll zudem möglichst vermieden werden und die Immunkonjugate sollen sich tierschutzgerecht und in großen Mengen preisgünstig herstellen lassen.

Eingangs der Erfindungsbeschreibung sollen nachstehend weitere Begriffe definiert werden, wie sie durchgehend in der folgenden Erfindungsbeschreibung und auch in den Patentansprüchen verstanden werden. Sie sollen im Rahmen dieser Anmeldung keine andere Bedeutung haben:

### "Radioimmunkonjugat":

Hierunter werden in dieser Anmeldung Verbindungen aus IgY-Präparationen von SPF-Hennen oder transgenen SPF-Hennen verstanden, die mit diagnostischen und/oder therapeutischen Radionuklide und/oder Verstärkermolekülen konjugiert sind und im Rahmen der nuklearmedizinischen Diagnostik und/oder Radioimmuntherapie angewendet werden.

### "Immuntoxin":

Es werden Verbindungen aus IgY-Präparationen verstanden, die mit Toxinen (pflanzliches Toxin, Zytostatikum, Chemotherapeutikum, Antibiotikum etc.) konjugiert sind und im Rahmen der Immuntoxintherapie angewendet werden.

### "Spezifisches Targeting":

Hiermit wird der Vorgang bezeichnet, bei dem der Signal- oder Wirkstoff durch das Immunglobulin an das gewünschte Zielantigen gebunden wird.

### "Pretargeting":

Umfasst hier Anwendungen, die eine Verstärkung von Signalen oder Wirkungen der IgY-Präparationen erzeugen, indem weitere Antikörper einer anderen Spezies oder andere geeignete Moleküle (hier auch unter dem Oberbegriff "Verstärker-Molekül" zusammengefasst) in irgendeiner Phase zwischen Antigenbindung und Signal- oder Wirkstoff zwischengeschaltet werden.

### "Chimäre Eidotter-Antikörper":

Es werden hiermit
i) humanisierte Immunglobuline, bei denen Fab-Fragmente vom Huhn mit Fc-Fragmenten von humanem IgG biochemisch miteinander verknüpft wurden, oder
ii) humanisierte Immunglobuline aus transgenen SPF-Hühnern, in denen die Genabschnitte für die konstanten Regionen von IgY mehr oder weniger vollständig durch die konstanten Regionen von humanem IgG ersetzt sind,

### "IgY-Konjugat":

Dieser Begriff umfasst hier Verbindungen aus polyklonalen IgY-Präparationen (immunologische Komponente) und Signalstoffen und/oder Wirkstoffen (diagnostische oder therapeutische Komponente) und/oder Verstärkermolekülen.

Die Aufgabe wird erfindungsgemäß gelöst durch polyklonale IgY-Konjugate aus intakten Eidotter-Antikörpern (IgY), IgY-Fragmenten, Fab-Konstrukten oder humanisierten Eidotter-Antikörpern vom SPF-Huhn, vorzugsweise vom transgenen SPF-Huhn. Die Antikörper bzw. Antikörperfragmente sollen als immunologisch wirksame Komponente mit mindestens einer weiteren Komponente konjugiert sein, wobei es sich um einen Signalstoff, einen Wirkstoff oder ein Verstärker-Molekül handeln kann.

Die Erfindung betrifft IgY-Konjugate aus Eidotter-Antikörpern (IgY) und deren Konfektionierung zu Diagnostika oder sowie zur experimentellen Anwendung im Tiermodell (beispielsweise Maus, Schaf oder nicht-humane Primaten). Ihre Anwendung ist nicht auf die parenterale Applikation beschränkt, sondern umfasst je nach Anwendungsziel auch äußerliche (z. B. beim Melanom) und enterale Applikationen (z. B. beim Ösophagus-Karzinom).

Die Erfindung betrifft außerdem die Konfektionierung der IgY-Konjugate als gebrauchsfertige Diagnostika oder Arzneimittel, entweder in Form der einzelnen Komponenten, als Markierungsbestecke (sog. Kits) oder als gebrauchsfertige Präparate. Als Zielantigene kommen alle Proteine, Peptide, Glykoproteine, Nukleinsäuren, Polysaccharide und Lipoproteine in Betracht. Die Erfindung betrifft vor allem IgY-Konjugate gegen Tumorantigene, Hormone, Rezeptorproteine, RNS- und DNS-Abschnitte, infektiöse Erreger, Prionen oder einen Teil der antigenen Determinanten dieser Zielantigene. Die erfindungsgemäßen IgY-Konjugate können dabei je nach Komplexität des für die Immunisierung verwendeten Antigens mehr oder weniger polyspezifisches IgY enthalten.

Ein weiterer Aspekt der vorliegenden Erfindung betrifft Pretargeting-Methoden, wobei entweder
i) biotinyliertes IgY an das Zielantigen bindet und als Vermittler für markiertes Avidin oder Streptavidin dient, oder
ii) unkonjugiertes IgY an das Zielantigen bindet und als Vermittler für humane oder humanisierte anti-IgY-Konjugate dient.
Im Fall (i) wird die starke Bindung zwischen Biotin und Avidin oder Streptavidin ausgenutzt, weshalb die Biotin-Komponente in dem IgY-Konjugat auch Verstärker-Molekül bezeichnet werden kann.

Schließlich betrifft die vorliegende Erfindung auch Zubereitungen und Anwendungen der erfindungsgemäßen IgY-Konjugate, durch die ihre diagnostischen und/oder therapeutischen Eigenschaften verbessert werden, wie Kombinationen mit chirurgischen Eingriffen, Chemotherapeutika, Immunmodulatoren und Paraimmunitätsinducern.

Im Folgenden werden die in unmittelbarem Zusammenhang mit der Erfindung stehenden Komponenten, deren Funktionen, Wirkweisen und die Anwendung der erfindungsgemäßen IgY-Konjugate näher charakterisiert und die Lösungen ausführlich dargestellt:

### a) Immunologische Komponente

Erfindungsgemäß bestehen die immunologisch wirksamen Komponenten entweder aus
(i) intakten Eidotter-Antikörpern (IgY), IgY-Fragmenten, Fab-Konstrukten, oder
(ii) chimären Eidotter-Antikörpern, d. h. biochemisch humanisiertem IgY bzw. bevorzugt gentechnisch humanisiertem IgY aus Eiern transgener SPF-Hühner.
Dabei können die Fab-Konstrukte mono-, bi- oder trivalent sein und mono-, bi- oder trispezifisch wirken (d. h. eine, zwei oder verschiedene drei antigene Determinanten erkennen) Die Fab-Fragmente lassen sich durch enzymatische Verdauung erzeugen. Chimäre Antikörper können durch Verknüpfung des aviären Fragments mit dem Fc-Fragment eines anderen Organismus (hier vorzugsweise humanes Fc-Fragment) gewonnen werden. Humanisiertes IgY, bei dem nur noch die hypervariablen Regionen vom IgY des Huhns stammen und alle konstanten Regionen vom IgG des Menschen, kann in transgenen Hühnern erzeugt werden. Mit chimären Antikörpern lässt sich nicht nur die Bildung unerwünschter Antikörper gegen IgY reduzieren oder verhindern, sondern es lassen sich auch die natürlichen Effektor-Mechanismen der konstanten Regionen des humanen IgG nutzen.

### b) Signalstoffe

Die erfindungsgemäßen IgY-Konjugate enthalten als diagnostische Komponente Signalstoffe wie Radionuklide (Gamma-Strahler wie Technetium-99m, Indium-111, Iod-123, Iod-125, Iod-131, Thallium-201, Selen-75, Gallium-67, Xenon-133), Enzyme (wie Peroxidasen, Alkalische Phosphatasen und Galaktosidase), Photosensibilisatoren (Porphyrin-Derivate etc.) oder Farbstoffe (z.B. Fluorochrome, kolloidales Gold). Für die szintigraphische Diagnostik sind je nach Fragestellung, Applikationsweg, Affinität der Antikörper und Radionuklid Aktivitäten von 10 - 1850 MBq (0,27 - 50 mCi) erforderlich. Im einzelnen kommen z.B. für Iod-131 37 - 370 MBq (1 - 10 mCi) (bevorzugt 37 - 74 MBq (1 - 3 mCi)) in Betracht, für Iod-123 185 - 370 MBq (5 - 10 mCi), für Technetium-99m 74 - 1480 MBq (2 - 40 mCi) (bevorzugt 74 - 740 MBq (2 - 20 mCi)), für Indium-111 und Gallium-67 74 - 185 MBq (2 - 5 mCi) oder für Selen-75 um 10 MBq (0,27 mCi).

### c) Wirkstoffe

Die erfindungsgemäßen IgY-Konjugate können alternativ oder zusätzlich als therapeutische Komponente Radionuklide enthalten (Beta-Strahler, Alpha-Strahler oder Auger-Elektronen-Emitter), z.B. Phosphor-32, Strontium-89, Yttrium-90, Iod-125, Iod-131, Samarium-153, Erbium-169, Rhenium-186, Rhenium-188, Kr-85, Holmium-166, Astat-211, Wismuth-212, Wismuth-213, Radium-224, Actinium-225. Wie bei der Diagnostik hängen die benötigten Aktivitäten bei der Radioimmuntherapie von mehreren Faktoren ab: Art der Erkrankung, Strahlensensibilität der erkrankten Zellen, Masse der zu behandelnden Zellen, Ausdehnung und Verteilungsmuster der Erkrankung, Antigen- Expression, Applikationsweg, Affinität der Antikörper und Energiespektrum des Radionuklides. In Betracht kommen daher z.B. bei Jod-131 Aktivitäten in einem weiten Bereich von 370 MBq bis 11,1 GBq (10 - 300 mCi). Diese Aktivität wird im Allgemeinen einmalig appliziert, kann aber auch fraktioniert in Intervallen von mehreren Wochen bis Monaten (vorzugsweise 3 - 6 Wochen) bzw. bei Auftreten eines Rezidivs bis zu einer Gesamtdosis von 40 - 80 GBq (1 - 2 Ci) wiederholt verabreicht werden. Die Konjugation von tumorspezifischem IgY mit Zytostatika kann die Nachteile herkömmlicher Chemotherapien verringern. Zu den größten Problemen der Onkologie gehören die begrenzte diagnostische Sicherheit bei den Staging-Untersuchungen (besonders das Fehlen von Techniken, mit denen das Vorliegen von Mikrometastasen bewiesen oder ausgeschlossen werden kann) sowie die geringe therapeutische Breite der Zytostatika infolge ihrer hohen Toxizität bei gleichzeitig geringer Spezifität. Diese Faktoren führen immer wieder zu einer erheblichen Unsicherheit bezüglich des therapeutischen Procedere. Eine nebenwirkungsärmere, durch Antikörper vermittelte spezifische Zytostase würde die Entscheidung für eine Chemotherapie erleichtern und folglich aufwendige, teure und/oder belastende Staging-Untersuchungen einsparen helfen. Im Sinne der Erfindung können die Eidotter-Antikörper ebenso mit verschiedenen Zytostatika konjugiert werden (z. B. Alkylantien, Vinkaalkaloiden, interkalierende Antibiotika, Antimetabo-liten, Taxol). Die erfindungsgemäßen IgY-Konjugate können ebenso als therapeutisch wirksame Komponente Toxine enthalten (Ricin A, Abrin, Pseudomonas-Exotoxin, Diphtherie-Toxin, Gelonin u. a.). Auch Chemotherapeutika (Cytostatika, Antibiotika, antivirale Substanzen), Fibrinolytika (z. B. Streptokinase, Urokinase, rt-PA), Enzyme zur Aktivierung von Prodrugs (z. B. bakterielle Carboxypeptidase CPG2, die die hydrolytische Spaltung von reduziertem und nicht-reduziertem Folat katalysiert) oder Prodrugs selbst eignen sich als therapeutische Komponenten in den erfindungsgemäßen IgY-Konjugaten. Schließlich können die erfindungsgemäßen IgY-Konjugate photosensitive Substanzen als wirksame Komponente enthalten, die es ermöglichen, mit einer photodynamischen Therapie erkrankte Zellen gezielt zu zerstören. Photosensibilisatoren können z. B. Photofrin, 5-Aminolävulinsäure oder Foscan sein.

### d) Verstärker-Moleküle

Die erfindungsgemäß zu Diagnostika oder Therapeutika konfektionierten IgY-Konjugate können Biotin enthalten, um auf der Basis von Avidin-Biotin- oder Streptavidin-Biotin-Bindungen das Signal oder die Wirkung zu verstärken (sogenanntes Pretargeting). Darüber hinaus können Signal oder Wirkung der IgY-Konjugate durch weitere Komponenten verstärkt werden, die an das IgY-Molekül konjugiert werden (z. B. Komplement).

### e) Hilfsstoffe, komplementäre Maßnahmen

Die erfindungsgemäßen IgY-Konjugate können zur Optimierung des Therapieziels mit anderen Wirkstoffen sinnvoll kombiniert werden. Aviäre Antikörper können die Bildung von humanen anti-IgY-Antikörpern auslösen. Bei mehrfacher Anwendung von IgY-Konjugaten kommt es dann zu einer Neutralisierung der diagnostisch oder therapeutisch eingesetzten Antikörper oder gar zu allergischen Reaktionen. Dies kann durch eine Suppression des Immunsystems beispielsweise mit Cyclosporin verhindert werden. Andererseits kann der Einsatz von radioaktivem lod zu einer unerwünschten radioaktiven Belastung der Schilddrüse führen. Daher ist es in diesen Fällen sinnvoll, die Funktion der Schilddrüse vor einer Radioimmuntherapie mit IgY-Iod-131 durch Gabe von Kaliumiodid oder Natriumperchlorat zu blockieren. Bei einer Immuntherapie viraler Infektionen würden zudem freie Viruspartikel IgY-Konjugate abfangen, die dann nicht mehr für das spezifische Targeting Virus replizierender Zellen zur Verfügung stehen. Daher ist es sinnvoll, die virale Replikation durch eine Vorbehandlung mit einem oder mehreren antiviral oder antiretroviral wirkenden Chemotherapeutika zu hemmen und die erneute Antigen-Expression ggf. durch vorübergehendes Absetzen dieser Medikation zu triggern oder zu induzieren. Darüber hinaus könnte die Freisetzung der Viruspartikel durch Mittel, die die Zellmembran stabilisieren (z. B. Interferon-alpha), gehemmt werden. Bei latenten Virusinfektionen (z. B. HIV- oder EBV-Infektionen) kann es auf der anderen Seite erforderlich sein, die Antigen-Expression der infizierten Zellen zu stimulieren (z. B. durch Einsatz von Interleukin-2) oder die Effektivität der IgY-Konjugate durch andere antivirale Wirkstoffe (z. B. Interferon-alpha und/oder Ribavirin bei der Therapie der Hepatitis C) zu steigern. Ebenso kann es bei der Behandlung bakterieller Infektionen sinnvoll sein, komplementär weitere Wirkstoffe (Antibiotika) einzusetzen. Analog dazu kann es bei onkologischen Erkrankungen erforderlich sein, beispielsweise eine Radioimmuntherapie durch die Gabe von Cytostatika oder eine Immuntoxin-Therapie durch eine lokale externe Radiation zu ergänzen. Ganz allgemein können auch Immunmodulatoren oder Paraimmunitätsinducer den Effekt einer Radioimmuntherapie, Immuntoxintherapie oder photodynamischen Therapie günstig beeinflussen.

### f) Markierungsmethoden

Für die radioaktive Markierung von murinen und humanen monoklonalen Antikörpern stehen bereits seit Jahren zahlreiche etablierte Methoden zur Verfugung (Peters JH, Baumgarten H (Eds.): Monoclonal Antibodies. Springer-Verlag, Berlin 1992). Metallische Radionuklide wie Technetium-99m, Indium-111 oder Astat-211 werden meistens durch Komplexbildner - z. B. Diethylentriaminpentaessigsäure (DTPA) oder Deferoxamin (DFA) - oder durch partielle Reduktion der Disulfidbrücken des Antikörpers (z.B. mittels 2-Mercaptoethanol) stabil an Antikörper gebunden. Viele radioaktive Iod-Isotope können auch durch direkte elektrophile Substitution an aktivierte aromatische Gruppen der Antikörper (v. a. Phenolring des Tyrosins) konjugiert werden. Als Oxidationsmittel zur Erzeugung von Iodkationen werden in der Regel Kaliumiodat (KIO₃), gelöstes Chloramin T bzw. an Träger (z. B. Polystyrol-Perlen) gebundenes Chloramin T (Iodobeads) oder Iodogen (1,3,4,6-Tetrachlor-3α,6α-diphenyl-glycouril) eingesetzt. Jeder einzelne Antikörper kann mit mindestens einem radioaktiven Atom markiert werden. Um eine höhere spezifische Aktivität zu erzielen, ohne dass die spezifische Bindungsfähigkeit der Antikörper beeinträchtigt wird, kann die Konjugation in Gegenwart des Zielantigens erfolgen, das anschließend wieder abgetrennt wird, wodurch eine Bindung des Radionuklides im Bereich der hypervariablen Regionen verhindert wird. Fluorochrome, Photosensibilisatoren, Cytostatika, Toxine können mit Methoden an Antikörper konjugiert werden, die auf Reaktionen mit den Aminogruppen der Antikörper beruhen, z. T. auch über kommerziell erhältlich Spacer oder durch Einführung von SH-Gruppen in die Antikörper. Andere Methoden beruhen auf der Spaltung der Antikörper und Freisetzung von SH-Gruppen, an die die Signal- oder Wirkstoffe konjugiert werden können. Außerdem können die Kohlenhydratreste der Antikörper mit Periodat oxidiert werden, wodurch Aldehydgruppen entstehen, über die Signal- und Wirkstoffe konjugiert werden können. Schließlich können bi- und trifunktionale Fab-Konstrukte mit einem Wirkstoff an einen oder zwei Fab-Fragmente des Moleküls gebunden werden und über das freie Fab-Fragment an das Zielantigen andocken.

### g) Konfektion

Im Sinne der Erfindung können für Radioimmunkonjugate Markierungsbestecke (Präparations-Kits) mit IgY-Präparationen angeboten werden, so dass diese durch den Anwender vor Ort mit dem meist kurzlebigen Radionuklid markiert werden können. Bei ausreichend langer HWZ können auch die mit einem Radionuklid konjugierten IgY-Präparationen (d. h. fertige Radioimmunkonjugate) direkt an den Anwender geliefert werden. In den Markierungsbestecken bzw. fertigen Präparaten liegen die IgY-Präparationen oder fertigen Radioimmunkonjugate als sterile isotone Injektionslösung ggf. in geeignetem Puffer (vorzugsweise PBS, pH 7,4) mit geeigneten Zusätzen (z. B. Stabilisatoren) und in geeigneter Verpackung (Kunststoff-/Glasampulle, Durchstechflasche, ggf. im Bleibehälter, ggf. gekühlt bei +4 bis -20°C) vor. Bei Markierungsbestecken befindet sich die IgY-Präparation in der ersten Ampulle/Durchstechflasche) und in einer zweiten Ampulle/Durchstechflasche das Reduktionsmittel (z. B. bei Markierung mit Technetium-99m) bzw. das Oxidationsmittel (z. B. bei Markierung mit Iod-131). Für die Reinigung des Radioimmunkonjugats kann jedes Markierungsbesteck eine Chromatographie-Säule enthalten und zur Qualitätskontrolle beispielsweise ein Dünnschicht-Chromatographie-Set. Je nach Bedarf können die Markierungsbestecke die IgY-Präparationen oder die fertigen IgY-Konjugate für eine oder gleich mehrere Anwendungen enthalten.

Gemäß der Erfindung sind folgende Anwendungsbereiche und Applikationen vorgesehen:
a) Anwendungsbereiche allgemein: Die erfindungsgemäßen IgY-Konjugate können mit einer Vielzahl von Antigenen reagieren: mit physiologischen Molekülen, mit tumorspezifischen Antigenen, mit infektiösen Erregern und deren Antigenen, mit Prionen sowie mit RNS- oder DNS-Abschnitten oder den damit assoziierten Molekülen. Je nach Signal- oder Wirkstoff (Radionuklid, Enzym, Fibrinolytikum, Farbstoff, Photosensibilisator, Toxin) können die erfindungsgemäßen IgY-Konjugate zur in vitro- und in vivo-Diagnostik und/oder Therapie von Tumoren, Infektionen, Gerinnungsstörungen und Autoimmunkrankheiten verwendet werden.
b) Physiologische Zielantigene: Zu den physiologischen Molekülen, die als Target für die erfindungsgemäßen IgY-Konjugate in Betracht kommen, zählen die Leukozytenantigene entsprechend der CD-Klassifikation (Antigene der NK-Zellen, B-Zell-Antigene, myeloische Antigene, Progenitorantigene, Aktivierungsantigene, Adhäsionsmoleküle, Zytokinrezeptoren), sowie intrazelluläre Moleküle (z. B. Zytokeratine), Rezeptoren (Androgen-, Östrogen-, Dopamin-D2- oder Somatostatinrezeptoren usw.) oder beispielsweise Gerinnungsfaktoren (z. B. Fibrin oder Plasminogen).
c) Pathologische Zielantigene Zu den tumorspezifischen Antigenen, die als Target für IgY-Konjugate in Betracht kommen, zählen ebenfalls bestimmte überexprimierte Leukozytenantigene (z. B. CD20, CD22), überexprimierte Androgen- oder Östrogenrezeptoren, Calcitonin und Thyreoglobulin, aber auch, im engeren Sinne Tumormarker wie Egp 34, Ca 15-3, Sialyl-Le^{a}-Antigen, BCA 225, Melanoma associated antigens (MAA), CEA, 17-1A, PAP, Neuronen spezifische Enolase (NSE), terminale Desoxynukleotidyl-Transferase (TdT), Bromdesoxyuridin (BrdU), Ki 67, PCNA, Myeloperoxidase (MPO) und mutiertes p53. Zu den infektiösen Erregern, die das Target für die erfindungsgemäßen IgY-Konjugate bilden können, zählen Bakterien (Bacillus anthracis, Borrelien, Brucellen, Mycobacterien, Pseudomonas aeroginosa, Salmonellen, Staphylokokken, Toxoplasma gondii, Treponemen, Trypanosomen), Viren (HIV-1, HIV-2, HTLV-1, HTLV-2, HCV und andere Flaviviren, HBV, EBV, HSV, HHV-8, Coxsackieviren, Polioviren, Cytomegalo-Virus, Influenza-Viren, Rubellavirus, Papillomaviren, Dengueviren, Hantaviren, Arenaviren, Bunyaviren, Filoviren), Protozoen (Plasmodien, Toxoplasmen, Acanthamoeba), Mykoplasmen, Pilze (Candida, Aspergillus), Parasiten (Ascariden, Echinococcus, Leishmania, Loa-Loa, Onchocerca volvulus, Mansonella, Brugia-Filarien, Cysticercus, Schistosomen, Bancrofti-Filarie) und infektiöse Partikel (Prionen). Die antigenen Determinanten infektiöser Erreger können auch bei der Diagnostik und/oder Therapie von Tumoren als Target der erfindungsgemäßen IgY-Konjugate eine Rolle spielen, so bei Burkitt-Lymphomen, nasophariyngealen Karzinomen, M. Hodgkin, T-Zell-Lymphom und immunoblastischen Lymphomen, die sich durch Expression von EBVassoziierten Antigenen (EBNA 1-3, LMP 1-2) auszeichnen, beim Kaposi-Sarkom und AIDSassoziierten Lymphomen, die beide mit HHV-8 assoziiert sind, oder bei der adulten T-Zell-Leukämie (ATL), die mit der HTLV-I-Infektion assoziiert ist, oder bei Genitalkrebs, der mit HPV assoziiert sein kann.
d) Applikationsformen: Je nach Bedarf können die erfindungsgemäßen IgY-Konjugate in vitro oder in vivo angewendet werden. Die Anwendung kann in vivo sowohl äußerlich oder enteral (z. B. im Rahmen einer photodynamischen Therapie) als auch parenteral (beispielsweise bei Szintigraphie, SPECT, Radioimmuntherapie oder Immuntoxin-Therapie) erfolgen. Der Begriff parenteral umfasst hier intravenöse, intraarterielle, subcutane, intracutane, intrathecale, interstitielle, intracavitäre und intraläsionale oder intratumorale Applikationswege.

Die Erfindung wird im Folgenden anhand detaillierter Ausführungsbeispiele näher erläutert und beschrieben. Die den Zeichnungen und der Beschreibung zu entnehmenden Merkmale können bei anderen Ausführungsformen der Erfindung einzeln für sich oder zu mehreren in beliebigen Kombinationen Anwendung finden. Die nachfolgenden Anwendungen und Ausführungsarten stellen lediglich Beispiele dar, die in keiner Weise die Möglichkeiten der erfindungsgemäßen IgY-Konjugate auf der Basis des SPF-Huhns erschöpfend darstellen können. Der Fachmann wird daher weitere Ausführungen und Anwendungen ableiten, die unter die Ansprüche der vorliegenden Erfindung fallen.

Die Zeichnungen zeigen:

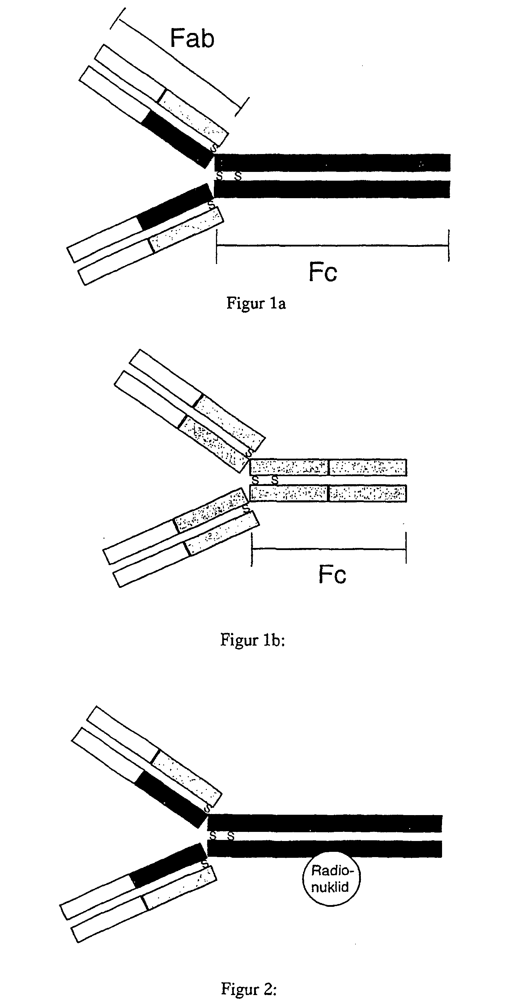

- Figur 1:: Immunglobulin vom Huhn und zum Vergleich vom Säuger
- Figur 1a:: IgY vom Huhn
- Figur 1b:: IgG vom Säuger inkl. Mensch
- Figur 2:: Radioimmunkonjugat auf der Basis von intaktem IgY
- Figur 3:: Radioimmunkonjugat auf der Basis eines monovalenten IgY-Fab-Fragments.

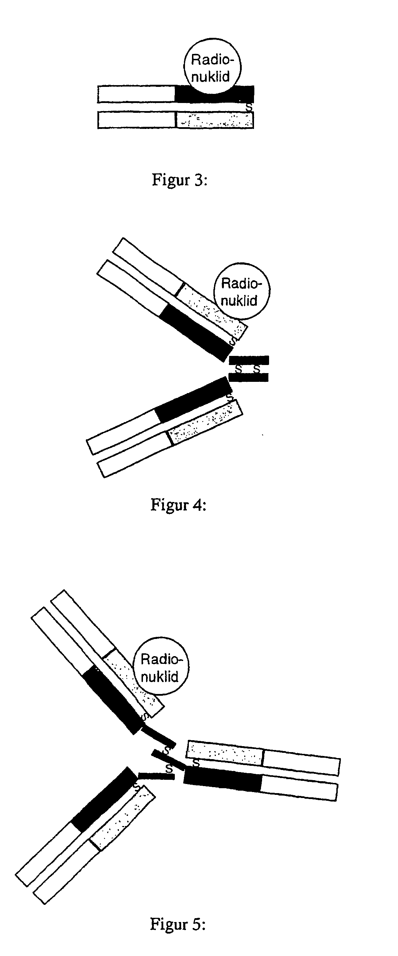

- Figur 4:: Radioimmunkonjugat auf der Basis eines bivalenten IgY-F(ab')₂-Fragments.
- Figur 5:: Radioimmunkonjugat auf der Basis von drei miteinander verknüpften IgY-Fab-Fragmenten (trivalentes Fab-Konstrukt).

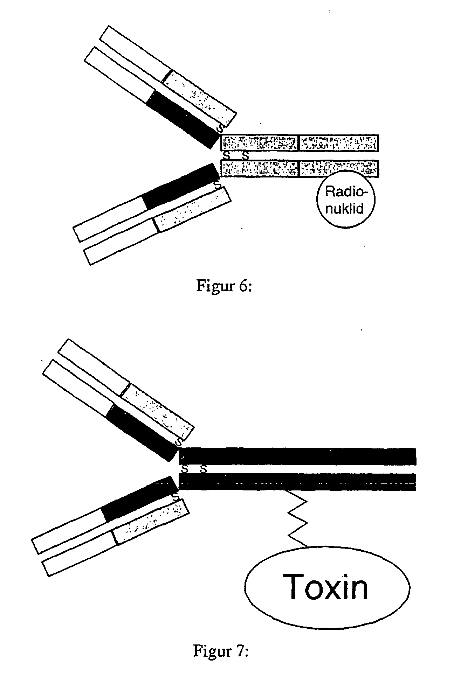

- Figur 6:: Radioimmunkonjugat auf der Basis von humanisiertem IgY (IgY-Fab + humanes Fc).
- Figur 7:: Immuntoxin auf der Basis von intaktem-IgY.

### Beispiel 1:

Zur Herstellung der erfindungsgemäßen IgY-Konjugate werden SPF-Hühner oder transgene SPF-Hühner vorzugsweise in der 14. Lebenswoche mit 50 - 1000 µg (vorzugsweise 50 - 200 µg) des Zielantigens bzw. eines Fragments davon und komplettem Freunds Adjuvans s.c. bzw. (vorzugsweise) i.m. immunisiert. Danach erfolgen verteilt über nahezu den gesamten Legezeitraum drei Boosterungen bis zum Erreichen des maximalen IgY-Titers und drei Auffrischungen mit jeweils 50-1000 µg (vorzugsweise 50-200 µg) desselben Antigens und inkomplettem Freunds Adjuvans zur Erhaltung des maximalen IgY-Titers. Die Injektionslösung enthält z.B. jeweils 1 ml Antigen-Suspension und 0,5 ml Adjuvans. Ein bevorzugtes Immunisierungsschema für Hühner mit SPF-Status hat demnach etwa folgenden zeitlichen Ablauf:

| | | | |
|---|---|---|---|
| Tag 0* | = 1. Immunisierung (Basisimmunisierung) | i.m.-Injektion | |
| Tag 28 | = 2. Immunisierung (1. Boosterung) | i.m.-Injektion | |
| Tag 56 | = 3. Immunisierung (2. Boosterung) | i.m.-Injektion | IgY-Gewinnung |
| Tag 84 | = 4. Immunisierung (3. Boosterung) | i.m.-Injektion | IgY-Gewinnung |
| Tag 168 | = 5. Immunisierung (1. Auffrischung) | i.m.-Injektion | IgY-Gewinnung |
| Tag 252 | = 6. Immunisierung (2. Auffrischung) | i.m.-Injektion | IgY-Gewinnung |
| Tag 336 | = 7. Immunisierung (3. Auffrischung) | i.m.-Injektion | IgY-Gewinnung |
| Tag 490 | = Nutzungsende für IgY-Gewinnung | | |

| | | | |
|---|---|---|---|
| * = 14. Lebenswoche | | | |

Während des Zeitraums von 12 - 14 Monaten lassen sich die Eier der unter SPF-Bedingungen gehaltenen Hühner sammeln und verarbeiten. Das Eigelb wird zunächst vom Eiweiß separiert und kann mit Standardmethoden nach Schade et al. (Chicken Egg Yolk Antibodies, Production and Application. Springer-Verlag, Berlin 2001 [1]) aufgearbeitet werden. Der entscheidende Vorteil der SPF-Hühner ist, dass diese Tiere infolge ihrer Abstammung von SPF-Hennen und der Haltung unter SPF-Bedingungen weder durch Immunisierungen noch durch spezifische Infektionskrankheiten immunologisch vorbelastet sind. Daher reagieren diese Tiere mit einer besonders intensiven Immunantwort gegen das Antigen, mit dem sie immunisiert wurden, so dass der Anteil spezifischer Antikörper um ein vielfaches höher liegt (50 - 90% spezifisches IgY) als bei Immunisierung konventioneller Hühner. Darüber hinaus kann bei diesen Tieren der hoher Titer spezifischer Antikörper nach dem Boostern durch mehrfache Auffrischungs-Immunisierungen bis zum 16. Lebensmonat stabil gehalten werden. Dies hat wesentliche Bedeutung für die erfindungsgemäßen IgY-Konjugate, denn es ist gut belegt, dass sich die Lymphozyten-Populationen durch eine wiederholte Stimulierung des immunologischen Gedächtnisses derart verändern, dass besonders hochaffine Antikörper gebildet werden. Daher unterscheiden sich die IgY-Präparationen aus Eiern von SPF-Hühnern auch hinsichtlich der Affinität zum spezifischen Antigen von IgY-Präparationen aus konventionellen Hühnern. Auf molekularer Ebene ist die höhere Affinität durch die Struktur der hypervariablen Region dieser Antikörper bedingt, die besser zum Antigen passt als die Struktur der hypervariablen Region weniger affiner Antikörper. Die erfindungsgemäßen IgY-Konjugate aus SPF-Hühnern unterscheiden sich daher letztendlich auf molekularer Ebene (d. h. durch die besondere Struktur ihrer hypervariablen Regionen) reproduzierbar von IgY-Konjugaten aus konventionellen Hühnern.

### Beispiel 2:

In diesem Beispiel wird die Herstellung von humanisiertem IgY aus transgenen Hühnern beschrieben. Die Transfektion kann in an sich bekannter Weise wie in US 5,162,215, US 6,020,465 und WO 00/75300 beschrieben erfolgen. Sie kann auch durch Mikroinjektion in die Spermien des Hahns erfolgen. Durch künstliche Befiuchtung wird das Merkmal dann in die Eizelle eingebracht und an die Nachkommen weitergegeben. Alternativ kann die Transfektion durch retrovirale Infektion der Spermien erfolgen. Dabei trägt ein replikationsdefizientes Retrovirus das humane Gen für das Fc-Fragment. Die Integration des Genabschnitts der konstanten humanen IgG-Domänen in den Immunglobulin-Genlocus des SPF-Huhns erfolgt durch homologe Rekombination. Der humane "knock-in" Gene Targeting Vektor trägt u. a. Sequenzen, die homolog sind zu den Exons des zu ersetzenden Gens, einen Selektionsmarker (z. B. Neomycin-Resistenz), die Exons der konstanten humanen IgG-Regionen und Promotoren. Durch transiente Cre-Rekombinase-Expression kann die Deletion der Neomycin- und Thymidinkinase-Kassette und der konstanten aviären IgY-Region erfolgen. Auf diese Weise entstehen transgene Hühner, die ihre Gene für humanisiertes IgY über die Keimbahn an ihre Nachkommen weitergeben. Die Selektion der transgenen Hühner kann durch Nachweis von IgY mit humanen konstanten Regionen im Blut oder in den Eiern der Hühner erfolgen. Zur Detektion der humanisierten Antikörper eignen sich ELISA, Western-Blot oder PCR.

### Beispiel 3:

Intakte Antikörper bedingen wegen ihrer langsamen Elimination aus dem Blut (HWZ: 1-3 Tage) bei der Immunszintigraphie oft lange Untersuchungszeiten bis zum Erreichen eines optimalen Kontrasts zwischen darzustellender Läsion und Hintergrund. Bei therapeutischen Anwendungen liegt das Problem eher darin, dass intakte Antikörper schlecht in solide Tumore oder jenseits der Blut-Hirn-Schranke gelangen. Aus diesem Grund bieten Konjugate auf der Basis von Fab-Fragmenten in bestimmten Situationen entscheidende Vorteile. Zur Herstellung der Fab-Konjugate werden SPF-Hühner oder transgene SPF-Hühner wie in Beispiel 1 geschildert mit 50 - 1000 µg (vorzugsweise 50 - 200 µg) des Zielantigens bzw. eines Fragments davon immunisiert. Die Eier werden wie oben angegeben aufgearbeitet und IgY per Affinitätschromatographie am spezifischen Antigen (Zielantigen) isoliert. Die intakten Antikörper werden dann enzymatisch z. B. mit Pepsin im Bereich der Hinge-Region gespalten, so dass Fab- und Fc-Fragmente entstehen. Die spezifischen Fab-Fragmente können per Affinitätschromatographie am spezifischen Antigen von Fc-Fragmenten und unspezifischen Fab-Fragmenten getrennt und isoliert werden. Die so erhaltenen spezifischen Fab-Fragmente werden steril filtriert. Sie können für die klinische Anwendung in einem Markierungsbesteck, das in einer zweiten Durchstechflasche das Reduktionsmittel für die Markierung enthält, zur Konjugation mit einem kurzlebigen diagnostischen Radionuklid (z. B. Technetium-99m) angeboten werden. Radioimmunkonjugate können mit längerlebigen diagnostischen oder therapeutischen Radionukliden (z. B. Iod-131) auch zentral beim Hersteller markiert und dann vertrieben werden. In diesem Fall erfolgt der Versand an den Anwender entsprechend den gültigen Richtlinien des Strahlenschutzes noch am Tag der Markierung, um einen Qualitätsverlust des Produkts durch Zerfall des Radionuklides und Dissoziation der Antikörper zu minimieren. Eine zentrale Markierung ist auch möglich, wenn die IgY-Präparation mit einem Chemotherapeutikum, Toxin, Fluorochrom, Fibrinolytikum, Enzym oder Prodrug konjugiert wird.

### Beispiel 4:

Radiologische Herde unklarer Dignität können heute mit Hilfe der Granulocyten-Szintigraphie differentialdiagnostisch abgeklärt werden. Die derzeit eingesetzten murinen monoklonalen Antikörper erkennen ein auf Promyelocyten, Myelocyten und Granulocyten exprimiertes Non-specific-cross-reacting-Antigen, entweder NCA-95 (= CD66b) oder NCA-90 (= CD66c). Sie werden mit diagnostischen Radionukliden (Gamma-Strahler) markiert und intravenös appliziert. Im Blut und am Entzündungsherd binden Sie an Granulocyten. Auf diese Weise können entzündliche Prozesse, z. B. eine Osteomyelitis, szintigraphisch nachgewiesen werden. Zur Herstellung der erfindungsgemäßen Radioimmunkonjugate werden SPF-Hühner mit NCA-90 oder NCA-95 bzw. Fragmenten davon, wie in Beispiel 1 beschrieben, immunisiert und geboostert. Das NCA-90- bzw. NCA-95-spezifische IgY wird wiederum affinitätschromatographisch an NCA-90 oder NCA-95 isoliert. Das sterile IgY kann dann als Markierungsbesteck, z. B. mit 1 mg IgY in, isotoner NaCl und einer zweiten Durchstechflasche mit Reduktionsmittel, für die Markierung mit Technetium-99m in den Handel gebracht werden. Das vom Anwender vor Ort präparierte anti-NCA-IgY-Tc-99m kann nach Abtrennung ungebundener Radionuklide über die mitgelieferte Säule und abschließender Qualitätskontrolle hinsichtlich der radiochemischen Reinheit des Endprodukts durch Hochleistungsflüssigkeitschromatographie (HPLC) oder vorzugsweise Dünnschicht-Chromatographie (DC) intravenös verabreicht werden. Im Gegensatz zu herkömmlichen monoklonalen Antikörpern, die nur eine antigene Determinate zu erkennen und binden in der Lage sind, werden mit den polyklonalen Eidotter-Antikörpern simultan verschiedene antigene Determinanten von NCA-95 oder NCA-90 erkannt, woraus eine bessere Bildgebung mit einem höheren spezifischen Uptake gegenüber dem Background resultiert.

### Beispiel 5:

IgY-Konjugate können auch zum Nachweis von Wächter-Lymphknoten verwendet werden. Ausgedehnte Lymphadenektomien, wie sie bei malignen Melanomen, Mamma- oder Prostata-Karzinomen erfolgen, sind im Allgemeinen mit einer hohen Morbidität verbunden. Daher ist der Nachweis einer Metastasierung in die im Abstromgebiet eines Tumors liegenden Lymphknoten eine für das therapeutische Procedere höchst wichtige Information. Bislang erfolgt er mit Tc-99m markiertem kolloidalem Humanalbumin oder mit Tc-99m-Nanokolloid. Die Partikelgröße liegt bei 100 - 1000 nm Durchmesser. Nach peritumoraler Injektion des Radiokolloids werden typischerweise in einem Zeitraum von 15 Minuten bis etwa sechs Stunden p.i. drei sequentielle planare Aufnahmen an der Gammakamera angefertigt. Der Nachteil dieser Methode liegt in der Partikelgröße und der fehlenden Tumorspezifität. Einerseits verbleibt dadurch ein großer Teil der Radioaktivität am Ort der Injektion und erschwert die Darstellung der Wächter-Lymphknoten durch Überstrahlung. Zum anderen werden Lymphknoten dargestellt, ohne dass ihr Befall dadurch bewiesen wäre. Werden hingegen in analoger Weise lymphozytenspezifische bzw. tumorspezifische Radioimmunkonjugate appliziert, so werden diese Nachteile teilweise oder ganz umgangen. Die gelösten IgY- oder Fab-Konjugate drainieren schneller über die Lymphbahnen als kolloidales Albumin, so dass weniger Restaktivität am Ort der Injektion verbleibt und die Wächter-Lymphknoten dadurch intensiver zur Darstellung kommen. Zu einer spezifischen Darstellung kommt es zudem dadurch, dass die Antikörper in den Lymphknoten spezifisch an Lymphozyten bzw. an metastasierte Tumorzellen binden. Diese Lymphknoten lassen sich auch intraoperativ mit einer geeigneten Gammasonde gezielt aufsuchen und dann resezieren. Alternativ oder zusätzlich zur Darstellung von Wächter-Lymphknoten kann eine weitgehend selektive Bestrahlung oder Chemotherapie dieser Lymphknoten bzw. der Lymphknoten-Metastasen erfolgen, wenn das diagnostisch verwendete IgY-Konjugat oder ein zweites IgY-Konjugat ein therapeutisches Radionuklid (z. B. I-131) oder ein Toxin (z. B. Taxol) enthält. Der Nachweis von Lymphknotenmetastasen eines Melanoms kann durch Radioimmunkonjugate gegen p210, einem Melanom assoziierten Antigen (MAA), erfolgen. Zur Herstellung der erfindungsgemäßen Radioimmunkonjugate werden SPF-Hühner mit 50 - 1000 µg (vorzugsweise 50 - 200 µg) p210 entsprechend Beispiel 1 immunisiert. IgY wird aus dem Eigelb isoliert. Das gereinigte IgY kann dann steril filtriert und lyophilisiert oder in steriler, isotoner PBS (pH 7,4) suspendiert werden. Das kommerziell verfügbare Präparationskit enthält z. B. in der ersten Durchstechflasche 100 mg spezifisches IgY und in der zweiten Durchstechflasche das Reduktionsmittel für die Markierung mit Tc-99m. Unmittelbar vor der geplanten Untersuchung wird die IgY-Präparation mit 10 - 1000 MBq (vorzugsweise 20 - 100 MBq) Tc-99m konjugiert. Nach peritumoraler Injektion des erfindungsgemäßen Radioimmunkonjugats erfolgen bis 24 Stunden p.i. (vorzugsweise drei bis sechs Stunden p.i. planare Aufnahmen in drei Ebenen unter der Gammakamera sowie ggf. eine ergänzende SPECT der betreffenden Region. Darüber hinaus können die betroffenen Lymphknoten anschließend intraoperativ mit einem geeigneten Handmessgerät detektiert und gezielt entfernt werden.

### Beispiel 6:

Die Diagnostik neuroendokriner Tumore erfolgt szintigraphisch mit Hilfe von Radioimmunkonjugaten. Phäochromozytome, Neuroblastome, Karzinoide und Paragangliome entstehen aus den Zellen des neuroendokrinen (APUD-) Systems, die über den gesamten Organismus verteilt sind. Sie besitzen daher im allgemeinen Somatostatin-Rezeptoren, durch die sie mit Hilfe von Indium-111 markiertem Octreotid (einem Somatostatin-Analogen) szintigraphisch dargestellt werden können. Somatostatin-Analoga binden jedoch an die Rezeptoren nur im Verhältnis 1:1. Polyklonales IgY erkennt hingegen mehrere antigene Determinanten des Rezeptors. Daraus resultieren ein besseres Targeting und eine sensitivere bildgebende Diagnostik. Zur Herstellung der IgY-Präparation gegen Somatostatin-Rezeptoren werden wiederum SPF-Hühner entsprechend Beispiel 1 immunisiert. Das spezifische IgY kann wie in Beispiel 3, 4 oder 5 in Form eines Markierungsbestecks angeboten werden. Es kann dann vom Anwender vor Ort mit einem Gamma-Strahler (z.B. Technetium-99m) markiert werden. 1 - 24 Stunden nach i.v.-Applikation von 100 mg spezifischem IgY-Tc-99m (10 - 1000 MBq, vorzugsweise 50 - 200 MBq) werden planare Aufnahmen in zwei Ebenen und ggf. eine SPECT durchgeführt. Die intraoperativ meist schlecht auffindbaren neuroendokrinen Tumoren können beim anschließenden operativen Eingriff mit einer geeigneten Sonde leicht lokalisiert und dann reseziert werden.

### Beispiel 7:

Eine weitere Anwendung der Radioimmunkonjugate ist die Behandlung von Non-Hodgkin-Lymphomen. Niedrig maligene Non-Hodgkin-Lymphome sprechen schlecht auf eine Chemotherapie an. Die Radioimmuntherapie hat sich in diesen Fällen als besonders wirksam erwiesen. In den meisten Studien wurden mit Iod-131 markierte murine monoklonale Antikörper gegen CD20 verwendet, ein Antigen, das auf der Zelloberfläche von 95% aller B-Zell-Lymphome exprimiert wird. Monoklonale Antikörper erkennen jedoch nur eine antigene Determinante dieses Antigens. Das Bindungsverhältnis beträgt daher nur 1:1. Ein besseres Targeting kann hingegen mit CD20-spezifischem IgY erzielt werden, das gleichzeitig mehrere Epitope auf dem Antigen erkennt. Dadurch wird auch die Strahlenbelastung des Knochenmarks reduziert, und es können höhere Strahlendosen appliziert werden, ohne dass eine Knochenmark-Transplantation erforderlich wird. Eine Verminderung der Strahlendosis kann auch durch Pretargeting-Methoden mit Avidin- oder Streptavidin-markierten monoklonalen Antikörpern und radioaktiv markiertem Biotin erreicht werden. Durch Pretargeting, wofür ebenfalls Schutz beantragt wird, kann allerdings auch der therapeutische Effekt einer Radioimmuntherapie mit IgY nochmals verbessert werden. SPF-Hühner werden hierfür mit 50 - 1000 µg (vorzugsweise 50 - 200 µg) CD20 wie in Beispiel 1 immunisiert. Über einen Legezeitraum von ca. 12 - 14 Monaten erhält man Eier, aus denen das Gesamt-IgY isoliert und an CD20 affinitätschromatographisch gereinigt wird. Für das Pretargeting wird das spezifische IgY steril filtriert, mit Avidin (oder Streptavidin) konjugiert und lyophilisiert oder ggf. mit weiteren Hilfsstoffen in isotoner NaCl (ggf. PBS, pH 7,4) gelöst. Das Markierungsbesteck enthält in diesem Fall vorzugsweise eine Durchstechflasche mit dem IgY-Avidin-Konjugat (100 µg - 5 mg, vorzugsweise 1 mg), eine zweite Durchstechflasche mit Biotin in lyophilisierter oder gelöster Form (1 - 1000 µg) und eine dritte Durchstechflasche mit einem Oxidationsmittel (z.B. Iodogen) für die Markierung mit Iod-131 (1 - 4 GBq), außerdem weitere Hilfsmittel wie Chromatographie-Säule und DC-Kit. Alternativ kann Biotin vom Hersteller zentral markiert werden und zusammen mit dem IgY-Avidin-Konjugat ausgeliefert werden. Zur Behandlung werden zunächst die IgY-Avidin-Konjugate intravenös appliziert, um die Lymphocyten zu markieren. Ein bis mehrere Tage später erfolgt die intravenöse Applikation des Iod-131-markierten Biotin. Alternativ kann die Radioimmuntherapie mit einem Alpha-Strahler wie Astat-211 durchgeführt werden.

### Beispiel 8:

Etwa die Hälfte aller Mamma-Karzinome besitzt Östrogen-Rezeptoren und ist hormonabhängig. Eidotter-Antikörper gegen Östrogenrezeptoren können daher nicht nur die Östrogenbindung an den Rezeptoren kompetitiv hemmen. Als Radioimmunkonjugat eignen sie sich auch zur Darstellung von Metastasen und zur internen Strahlentherapie. Für die Immunisierung der SPF-Hühner werden Peptide mit den extrazellulären antigenen Determinanten des Östrogenrezeptors verwendet. Die Immunisierung der SPF-Hühner sowie die Aufarbeitung der Eier und Reinigung des IgY können nach Beispiel 1 erfolgen, die Konfektionierung nach einem der anderen o. g. Beispiele.

### Beispiel 9:

Kommerziell erhältliche Antikörper wie URO7 erkennen Nierenzellkarzinome und binden nicht an gesundes Nierengewebe. Nierenzellkarzinome und ihre Metastasen können daher gezielt mit Radioimmunkonjugaten nachgewiesen und/oder behandelt werden. Um ein geeignetes Antigen zu identifizieren, werden die Proteine von homogenisierten Nierenkarzinomzellen elektrophoretisch aufgetrennt und isoliert. SPF-Hühner werden mit diesen Antigenen immunisiert und die gewonnenen Eidotter-Antikörper mit Nieren-Karzinomzellen und physiologischen Geweben hinsichtlich Spezifität und Kreuzreaktivität untersucht. Die selektierten Antikörper, die ausschließlich maligne Zellen erkennen, werden mit einem therapeutischen Radionuklid wie Iod-131 oder einem Zellgift wie Ricin A konjugiert und therapeutisch eingesetzt.

### Beispiel 10:

Eine weitere Anwendung der Radioimmunkonjugate wird in der Behandlung von HIV-Infektionen gesehen. Bei einer HIV-Infektion wird das retrovirale Genom in die Chromosomen der Wirtszelle eingebaut. Mit herkömmlichen Replikationshemmern kann die HIV-Infektion nicht geheilt werden. Die HIY replizierende Wirtszelle exprimiert dann HIV-Strukturproteine (p24, gp120 und gp41) auf ihrer Zelloberfläche. Antikörper gegen diese Strukturproteine erkennen diese Zellen und binden daran. Sind sie mit einem Radionuklid oder einem anderen Zellgift konjugiert, so können sie diese Zellen und damit die Produktionsorte von HIV gezielt zerstören. Voraussetzung für ein effektives Targeting der Zellen ist, dass die Zahl freier Viren im Blut beispielsweise durch eine komplementäre Replikationshemmung mit antiretroviralen Medikamenten reduziert wird. Damit eröffnen sich neuartige Möglichkeiten, die HIV-Infektion effektiv zu behandeln. Aus den eingangs genannten Gründen bietet die Verwendung polyklonaler Konjugate aus spezifischem IgY von SPF-Hülmern Vorteile gegenüber monoklonalen Antikörpern, wie sie in DE 198 09 785 beschrieben sind. Zur Gewinnung der Antikörper werden SPF-Hühner mit HIV p24, HIV-1 gp120 oder HIV-1 gp41 wie in Beispiel 1 immunisiert. Über einen Legezeitraum von rund 12 - 14 Monaten werden die Eier der unter definierten Bedingungen gehaltenen SPF-Hühner gesammelt. Das Eigelb wird vom Eiklar abgetrennt und das IgY nach einem der Standardverfahren isoliert. HIV spezifische Antikörper werden per Affinitätschromatographie am spezifischen Antigen gereinigt, so daß unspezifisches IgY abgetrennt wird. Die sterilen IgY-Präparation werden mit einem therapeutischen Radionuklid (z. B. Iod-131 oder Astat-211) konjugiert. Üblicherweise werden z. B. pro therapeutischer Anwendung 100 µg spezifisches IgY mit 1 - 4 GBq Iod-131 markiert. Im Falle der HIV-Infektion können sich u. a. wegen der relativ geringen Zahl infizierter Zellen (im Vergleich zur Zahl maligne entarteter Zellen bei Lymphomen) bereits wesentlich geringere Aktivitäten als therapeutisch wirksam erweisen. Die Markierung kann mit verschiedenen etablierten Methoden erfolgen. So könnte IgY-Iod-131 bevorzugt nach der Iodogen-Methode hergestellt werden. Sowohl die Ausbeute nach Konjugation des Radioimmunkonjugats als auch die Reinheit des Radioimmunkonjugats nach Abtrennung ungebundener Radionuklide können mit Hilfe von HPLC oder DC kontrolliert werden. Das unter sterilen Kautelen hergestellte und/oder sterilfiltrierte Radioimmunkonjugat kann als isotone Lösung infundiert werden.

### Beispiel 11:

Des Weiteren sollen IgY-Konjugate zur Behandlung von Autoimmun-Krankheiten verwendet werden. CD4-positive T-Zellen spielen eine wichtige Rolle in der Pathogenese von Autoimmun-Krankheiten und bei Abstoßungsreaktionen nach Organtransplantation. Monoklonale Antikörper gegen den CD4-Rezeptor zeigten bei M. Crohn, Lupus Erythematodes, Rheumatoider Arthritis und nach Hauttransplantation bei Mäusen jedoch klinisch nur geringe Effekte. Um eine effektive Depletion von CD4-positiven Zellen zu bewirken, können die Antikörper mit einem therapeutischen Radionuklid, einem Cytostatikum oder einem Toxin konjugiert werden. SPF-Hühner werden entsprechend Beispiel 1 gegen CD4 oder CD4-Fragmente immunisiert, Gesamt-IgY wird aus dem Eidotter isoliert, und das CD4-spezifische IgY kann durch Affinitätschromatographie an CD4 gereinigt werden. Gegebenenfalls werden die Antikörper enzymatisch gespalten und die aviären Fc-Fragmente durch humane Fc-Fragmente ersetzt. Die IgY-Präparation kann dann für die (vorzugsweise intravenöse) klinische Anwendung unkonjugiert oder als erfindungsgemäßes IgY-Konjugat mit einem Radionuklid markiert vertrieben werden.

### Beispiel 12:

Der Einsatz von IgY-Konjugaten zur Behandlung von Thrombosen stellt eine weitere Anwendung dar. Thrombosen werden im allgemeinen mit systemisch applizierten Fibrinolytika (Streptokinase, Urokinase oder rt-PA) behandelt. Streptokinase wird durch körpereigene Proteasen rasch abgebaut. Urokinase und rt-PA werden dagegen schnell in der Leber metabolisiert. Daher ist es sinnvoll, Fibrinolytika an den Thrombus zu fixieren (Bode et al., J Biol Chem 1989;264(2):944-8; Lijnen et al. Thromb Res 1990;57(3):333-42). SPF-Hühner oder transgene SPF-Hühner werden gegen humanes Fibrin in der in Beispiel 1 genannten Art und Weise immunisiert. Das spezifische IgY wird isoliert und gereinigt. Ggf. werden die Antikörper enzymatisch gespalten und die Fc-Fragmente abgetrennt. Intakte Antikörper oder Fab-Fragmente (5 - 50 mg) werden dann mit rt-PA (5 - 50 mg) vorzugsweise im Verhältnis 1:1 konjugiert. Diese IgY-Konjugate können lyophilisiert oder in steriler Lösung für Injektionszwecke in geeigneten Packungsgrößen für die Behandlung tiefer Beinvenenthrombosen, Lungenembolien, cerebrovaskulärer Insulte oder Myokardinfarkte angeboten werden. Alternativ kann eine zweite Gruppe SPF-Hühner gegen rt-PA (vorzugsweise eine Region außerhalb des aktiven Zentrums) immunisiert werden. Die Fab-Fragmente der ersten Gruppe können mit den Fab-Fragmenten der zweiten Gruppe hybridisiert werden. Auf diese Weise können fibrinspezifische bifunktionale Fab-Konstrukte erzeugt werden, an die über einen Arm rt-PA konjugiert ist.

### Beispiel 13:

Für die photodynamische Diagnostik oder Therapie werden IgY-Präparationen mit Photosensibilisatoren konjugiert. Die photodynamische Therapie stellt ein minimalinvasives Verfahren v. a. zur Behandlung von oberflächlichen Haut- und Schleimhautkarzinomen, Kaposi-Sarkomen und Melanomen dar. Sie beruht auf der tumorselektiven Anreicherung von lichtempfindlichen Substanzen, die ihre photodynamische Wirkung (Fluoreszenz bzw. Bildung zellschädigender Radikale und Zerstörung der Tumorvaskularisierung) durch Absorption von Licht entfalten. Der Erfolg der photodynamischen Therapie hängt entscheidend von der relativen Verteilung des Photosensibilisators im malignen und gesunden Gewebe ab. Die Fixierung des Photosensibilisators an die Tumorzellen kann beispielsweise bei Ösophaguskarzinomen oder malignen Melanomen durch tumorspezifische Antikörper erheblich verstärkt werden. Mit einer geeigneten Lichtquelle können dann Diagnostik oder Therapie erfolgen. Beim diagnostischen Einsatz kommt es unter violettem Licht zur spezifischen Fluoreszenz der maligne entarteten Zellen. Bei der photodynamischen Therapie wird maligne entartetes Gewebe unter der Einwirkung von rotem Licht nekrotisch.

### Literatur:

[1] Schade et. al.: Chicken Egg Yolk Antibodies, Production and Application. Springer Lab Manual, Berlin 2001);
[2] Hansen et al., J Immunol Methods 1998, 215(1-2): 1-7;
[3] Bouhours et al., Glycoconj J 1998, 15(1):93-9;
[4] Gassmann et al, FASEB J 1990, 4(8): 2528-32).

## Patentansprüche

1. IgY-Konjugate zur Herstellung eines Diagnostikums oder Therapeutikums, für nichtinfektiöse Entzündungen, Infektionskrankheiten, Gerinnungsstörungen, Autoimmunkrankheiten und onkologischen Erkrankungen bestehend aus einer polyklonalen IgY-Präparation aus Eiern von spezifiziert pathogenfreien (SPF)-Hühnem gemäß Europäischer Pharmakopoe und DAB10 oder bestehend aus einer polyklonalen IgY-Präparation aus Eiern von transgenen SPF-Hühnern, wobei die IgY-Konjugate polyklonales IgY mit konstanten Regionen von humanem IgG enthalten und weiterhin bestehend aus mindestens einem Signal- und/oder Wirkstoff und/oder Verstärker-Molekül.

2. IgY-Konjugate nach Anspruch 1, wobei die polyklonale IgY-Präparation gereinigte Eidotter-Antikörper (IgY), IgY-Fragmente, Fab-Konstrukte oder chimäre Eidotter-Antikörper sind.

3. IgY-Konjugate nach Anspruch 1, **dadurch gekennzeichnet, dass** die für die Zubereitung verwendeten IgY-Präparationen humanisierte Eidotter-Antikörper enthalten, die durch chemische Verknüpfung von variablen Regionen aviärer Antikörper mit konstanten Regionen von humanem IgG erzeugt sind.

4. IgY-Konjugate nach Anspruch 1 und 2, **dadurch gekennzeichnet, dass** die IgY-Präparation aus mono- oder bivalenten Antikörperfragmenten, vorzugsweise Fab oder F(ab)₂, besteht.

5. IgY-Konjugate nach Anspruch 1 und 2, **dadurch gekennzeichnet, dass** die IgY-Präparation aus bi-, tri- oder polyspezifischen Fab-Konstrukten besteht.

6. IgY-Konjugate nach Anspruch 1, **dadurch gekennzeichnet, dass** der Signalstoff ein diagnostisches Radionuklid, ein Enzym, ein Farbstoff und/oder ein Photosensibilisator ist.

7. IgY-Konjugate nach Anspruch 1, **dadurch gekennzeichnet, dass** der Wirkstoff ein therapeutisches Radionuklid, ein Zytostatikum, ein Toxin, ein Chemotherapeutikum, ein Prodrug, ein Enzym, ein Photosensibilisator und/oder ein Fibrinolytikum ist.

8. IgY-Konjugate nach Anspruch 1, **dadurch gekennzeichnet, dass** die IgY-Präparation mit einem Verstärker-Molekül, vorzugsweise mit Biotin oder einem Protein des Komplement-Systems konjugiert ist.

9. IgY-Konjugate nach einem oder mehreren der vorhergehenden Ansprüche, **dadurch gekennzeichnet, dass** der Signal- oder Wirkstoff über kovalente Bindung oder mittels eines Hilfsstoffs wie eines Chelatbildners, eines Verstärker-Moleküls und/oder eines für das Verstärker-Molekül hochaffinen Moleküls an die IgY-Präparation oder über die variable Region eines oder mehrerer Arme eines bi-, tri- oder polyspezifischen IgY-Konstrukts gebunden ist.

10. IgY-Konjugate nach einem oder mehreren der vorhergehenden Ansprüche, **dadurch gekennzeichnet, dass** die IgY-Präparation spezifisch ist für Tumorantigene, für Hormone, für RNAund/oder DNA-Abschnitte, für infektiöse Erreger oder ihre (Oberflächen-)Antigene, für Leukozyten-Antigene, für intrazelluläre Moleküle, für Rezeptor-Moleküle oder für Gerinnungsfaktoren.

11. IgY-Konjugate nach einem oder mehreren der vorhergehenden Ansprüche, wobei die Immunkonjugate im Sinne eines Pretargeting erst in vivo nach Bindung an das spezifische Antigen vorzugsweise über Biotin/Avidin oder Biotin/Streptavidin mit dem Signal- oder Wirkstoff konjugiert werden.

12. IgY-Konjugate nach einem oder mehreren der vorhergehenden Ansprüche, wobei die Immunkonjugate im Sinne eines Pretargeting erst in vivo nach Bindung an das spezifische Antigen über sekundäre humane anti-IgY-Antikörper mit dem Signal- oder Wirkstoff bzw. Verstärkermolekül konjugiert werden.

13. Konfektionierung von IgY-Konjugaten wie in den Ansprüchen 1 bis 12 beschrieben, wobei diesen mindestens ein Set für die Qualitätskontrolle per Dünnschicht-Chromatographie, Trennsäulen, Ständer für die Apparatur, Wasch- und Elutionspuffer beigegeben werden.

14. Konfektionierung nach Anspruch 13, wobei das IgY-Konjugat in lyophilisierter Form oder Suspension in Verbindung mit Zusatzstoffen wie Stabilisatoren, eingestellt auf einen optimalen pH-Wert, bereitgestellt wird.

15. Konfektionierung von IgY-Konjugaten nach Anspruch 13, wobei eine Applikationsvorrichtung beigegeben wird.

## Claims

1. IgY conjugates for producing a diagnostic agent or therapeutic agent for noninfectious inflammations, infectious diseases, coagulation disturbances, autoimmune diseases and oncological diseases, comprising a polyclonal IgY preparation derived from eggs of specific pathogen-free (SPF) hens in accordance with the European Pharmacopoeia and DAB10 or comprising a polyclonal IgY preparation derived from eggs of transgenic SPF hens, with the IgY conjugates containing polyclonal IgY possessing constant regions of human IgG, and furthermore comprising at least one signal compound and/or active compound and/or amplifying molecule.

2. IgY conjugates according to Claim 1, wherein purified egg yolk antibodies (IgY), IgY fragments, Fab constructs or chimeric egg yolk antibodies are the polyclonal IgY preparation.

3. IgY conjugates according to Claim 1, **characterized in that** the IgY preparations used for the formulation contain humanized egg yolk antibodies which are generated by chemically linking variable regions of avian antibodies to constant regions of human IgG.

4. IgY conjugates according to Claims 1 and 2, **characterized in that** the IgY preparation comprises monovalent or bivalent antibody fragments, preferably Fab or F(ab)₂.

5. IgY conjugates according to Clai ms 1 and 2, **characterized in that** the IgY preparation comprises bispecific, trispecific or polyspecific Fab constructs.

6. IgY conjugates according to Claim 1, **characterized in that** the signal compound is a diagnostic radio - nuclide, an enzyme, a dye and/or a photosensitizer.

7. IgY conjugates according to Claim 1, **characterized in that** the active compound is a therapeutic radionuclide, a cytostatic agent, a toxin, a chemotherapeutic agent, a prodrug, an enzyme, a photosensitizer and/or a fibrinolytic agent.

8. IgY conjugates according to Claim 1, **characterized in that** the IgY preparation is conjugated to an amplifying molecule, preferably to biotin or a protein of the complement system.

9. IgY conjugate according to one or more of the preceding claims, **characterized in that** the signal compound or active compound is bonded to the IgY preparation by way of a covalent bond by means of an auxiliary substance, such as a chelating agent, an amplifying molecule and/or a molecule which has a high affinity for the amplifying molecule, or by way of the variable region of one or more arms of a bispecific, trispecific or polyspecific IgY construct.

10. IgY conjugates according to one or more of the preceding claims, **characterized in that** the IgY preparation is specif ic for tumour antigens, for hormones, for RNA segments and/or DNA segments, for infectious pathogens or their (surface) antigens, for leukocyte antigens, for intra-cellular molecules, for receptor molecules or for coagulation factors.

11. IgY conjugates a ccording to one or more of the preceding claims, wherein the immunoconjugates are only conjugated, in the sense of a pretargeting, to the signal compound or active compound, preferably by way of biotin/avidin or biotin/streptavidin, in vivo following bindi ng to the specific antigen.

12. IgY conjugates according to one or more of the preceding claims, wherein the immunoconjugates are only conjugated, in the sense of a pretargeting, to the signal compound or active compound and/or amplifying molecule, by way of secondary human anti-IgY antibodies, in vivo following binding to the specific antigen.

13. Formulation of IgY conjugates as described in Claims 1 to 12, wherein at least one set for quality control, by means of thin layer chromatography, separating columns, stands for the equipment, and washing and elution buffers, are included with the conjugates.

14. Formulation according to Claim 13, wherein the IgY conjugate is provided in lyophilized form or in suspension in combination with additives such as stabilizers and adjusted to an optimum pH.

15. Formulation of IgY conjugates according to Claim 13, wherein an administration device is included.

## Revendications

1. Conjugués d'IgY pour l'établissement d'un diagnostic ou d'une thérapie d'inflammations non infectieuses, de maladies infectieuses, de troubles de la coagulation, de maladies auto immunes et de maladies oncologiques, constitués d'une préparation d'IgY polyclonale d'oeufs de poules (SPF) spécifiques exemptes de pathogène selon la pharmacopée européenne et DAB 10, ou constitués d'une préparation d'IgY polyclonale d'oeufs de poules SPF transgéniques, les conjugués d'IgY contenant de l'IgY polyclonale qui présente des régions constantes d'IgG humaine et étant en outre constitués d'au moins une molécule de signalisation et/ou active et/ou d'amplification.

2. Conjugués d'IgY selon la revendication 1, dans lesquels la préparation d'IgY polyclonale est constituée d'anticorps (IgY) purifiés de jaune d'oeuf, de fragments d'IgY, de constructs de Fab ou d'anticorps chimériques de jaune d'oeuf.

3. Conjugués d'IgY selon la revendication 1, **caractérisés en ce que** les préparations d'IgY utilisées pour la préparation contiennent des anticorps humanisés de jaune d'oeuf qui sont créés par ombinaison chimique de régions variables d'anticorps aviaires avec des régions constantes d'IgG humaine.

4. Conjugués d'IgY selon les revendications 1 et 2, **caractérisés en ce que** la préparation d'IgY est constituée de fragments d'anticorps mono ou bivalents, de préférence de fragments Fab ou F(ab)₂.

5. Conjugués d'IgY selon les revendications 1 et 2, **caractérisés en ce que** la préparation d'IgY est constituée de constructs bi-, tri- ou polyspécifiques de Fab.

6. Conjugués d'IgY selon la revendication 1, **caractérisés en ce que** la substance de signalisation est un radionucléide de diagnostic, une enzyme, un colorant et/ou un agent photosensibilisateur.

7. Conjugués d'IgY selon la revendication 1, **caractérisés en ce que** lasubstance active est un radionucléide thérapeutique, un cytostatique, une toxine, un agent de chimiothérapie, un précurseur de médicament, une enzyme, un agent photosensibilisateur et/ou un agent fibrinolytique.

8. Conjugués d'IgY selon la revendication 1 **caractérisés en ce que** la préparation d'IgY est conjuguée avec une molécule d'amplification, de préférence avec de la biotine ou une protéine du système complémentaire.

9. Conjugués d'IgY selon l'une ou plusieurs des revendications précédentes, **caractérisés en ce que** la substance de signalisation ou active est liée à la préparation d'IgY par liaison covalente ou au moyen d'une substance auxiliaire, par exemple un agent chélatant, une molécule d'amplification et/ou une molécule qui présente une forte affinité vis-à-vis de la molécule d'amplification ou par l'intermédiaire de la région variable d'une ou de plusieurs branches d'un construct bi-, tri- ou polyspécifique d'IgY.

10. Conjugués d'IgY selon l'une ou plusieurs des revendications précédentes, caracérisés en ce que la préparation d'IgY est spécifiquement prévue pour des antigènes tumoraux, des hormones, des fragments d'ARN et/ou d'ADN, des agents infectieux ou leurs antigènes (de surface), des antigènes de leucocytes, des molécules intracellulaires, des molécules de récepteursou des facteurs de coagulation.

11. Conjugués d'IgY selon l'une ou plusieurs des revendications précédentes, dans lesquels dans le cadre d'un ciblage préalable, les conjugués immunitaires ne .sont conjugués in vivo à la substane de signalisation ou active qu'après liaison à l'antigène spécifique, de préférence par l'intermédiaire de biotine/avidine ou de biotine/streptavidine.

12. Conjugués d'IgY selon l'une ou plusieurs des revendications précédentes, dans lesquels dans le cadre d'un ciblage préalable, les conjugués immunitaires ne sont conjugués in vivo à la substance de signalisation ou active ou bien à la molécule d'amplification qu'après liaison à l'antigène spécifique par l'intermédiaire d'anticorps anti-IgY humains secondaires.

13. Préparation de conjugués d'IgY selon les revendications 1 à 12, dans laquelle on ajoute à ces conjugués au moins une trousse de contrôle de qualité par chromatographie en couche mince, une colonne de séparation, des statifs pour les appareillgaes ainsi que des tampons de lavage et d'élution.

14. Préparation selon la revendication 13, dans laquelle le conjugué d'IgY est préparé sous forme lyophilisée ou d'une suspension associée à des additifs tels que des agents de stabilisation et ajustée à nue valeur optimale du pH.

15. Préparation de conjugués d'IgY selon la revendication 13, dans laquelle on ajoute un dispositif d'application.
